# EUROPEAN PATENT SPECIFICATION

(11) **EP 4 266 990 B1**
(45) Date of publication and mention of the grant of the patent: **19.11.2025**
(21) Application number: 21914869.9
(22) Date of filing: 27.12.2021
(51) Int. Cl.: A61B 5/00, A61B 5/05, A61B 1/00, A61B 6/00, A61B 10/00

(54) **MULTIFUNCTIONAL DEVICE AND METHODS FOR IMAGING SURFACE/VOLUME IRREGULARITIES**
MULTIFUNKTIONSVORRICHTUNG UND VERFAHREN ZUR ABBILDUNG VON OBERFLÄCHEN-/VOLUMENUNREGELMÄSSIGKEITEN
DISPOSITIF MULTIFONCTIONNEL ET PROCÉDÉS D'IMAGERIE D'IRRÉGULARITÉS DE SURFACE/VOLUME

(30) Priority: 28.12.2020 US 202063130935 P
(43) Date of publication of application: 01.11.2023
(73) Proprietor: Patternox Ltd., 1804343 Afula (IL)
(72) Inventor: AHARON, Ofir, 1804356 Afula (IL)
(74) Representative: Boult Wade Tennant LLP
(86) International application number: PCT/IL2021/051539
(87) International publication number: WO 2022/144879

(56) References cited:
- US-A1- 2008 049 433
- US-A1- 2012 113 506
- US-A1- 2013 307 950
- US-A1- 2018 325 377
- US-B2- 7 986 987
- US-B2- 9 338 379

## Description

### BACKGROUND

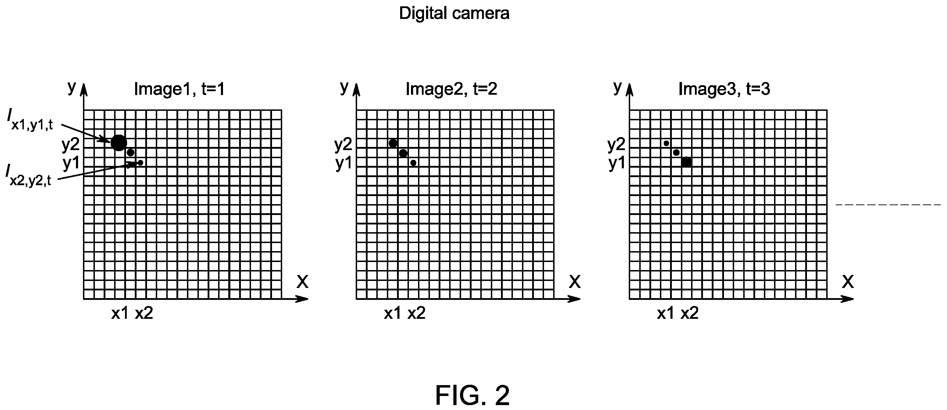

The present disclosure is directed in general to systems, methods and programs for generating an image of a scattering surface and/or volume portion of a subject depicting the irregularities of the scattering surface and/or volume irregularities **(SViR)** portion as a function of the scattering surface's and/or volume's density, as well as a function of the changes in refraction index irregularities and less as a function of color. More specifically, the disclosure relates to a method of imaging a scattering surface and/or volume implementable in a system having a radiation source **(RS)** operable to illuminate the scattering surface and/or volume portion from different states of illuminations, such as, for example, angular (azimuth and/or latitude, and/or distance from the portion of the at least one of: the tissue surface, and the tissue volume along the longitudinal axis *Z_{L}*) position changes of RS's Poynting Vector **(PV)** (see e. g., FIG.s 1A-1C), and collect a plurality of images for each state of PV. Due to the effect generated by the skewed illumination position on the SViR of the scattering surface and/or volume portion, each image will have different shadow for each state of illumination (e.g., angle of PV) (see e.g., FIG. 2).

Skin, and mainly skin lesion, is but one example of the scattering surface and/or volume portion. Malignant melanoma is one of the most rapidly increasing cancers in the world. Successful treatment of melanoma depends on early detection by clinicians with subsequent surgical removal of tumors. In recent years, considerable effort has been expended on developing reliable optical methods for characterizing tissue and monitoring changes. Optical scanners for melanoma detection, were approved for marketing. As reported, the 5-year survival rate is 93 to 97% for melanoma detected at an early stage (e.g., Melanoma in-situ), but drops to between 10 and 20% for advanced stage detection, implying that there is a need for accurate diagnostic devices to enable early detection while avoiding unnecessary biopsies.

For example, dermascopes utilizing light or Raman spectroscopy (for signal analysis) to distinguish malignant from benign skin lesions and have shown sensitivities (subjected to physician experience) ranging from 90 to 99%. Other types of dermascopes illuminate the skin at several wavelengths, and measures light scattered back from the skin (with or without polarizers), and use algorithms of color image analysis, combined with a skin disorder database, to provide treatment suggestion. Yet other devices are based on using a handheld, multispectral scanner and computer software to provide dermatoscopic images, dermal and epidermal pathological characteristics, as well as the ability to catalogue, monitor, and compare lesions over time. In a randomized controlled trial involving large number (over 1000) of suspicious pigmented lesions it was found that adding these instruments to best practices resulted in lower agreement, with inter- and intra-observer concordance being very low, even for expert observers' assessment that the lesion was benign, led to a higher proportion of referrals and therefore biopsies.

Despite extensive research in investigating the varied presentations and physical characteristics of melanoma (and other neoplasial pathologies), the clinical diagnostic specificity and selectivity remains suboptimal. Therefore, there is a need for a handheld, portable device that able to provide SViR imaging at early stages of the scattering surface and/or volume portion deformation monitoring changes over time.

### SUMMARY

In certain exemplary implementations, provided herein, are methods of imaging and characterizing a scattering surface and/or volume portion (e.g., a skin portion) implementable in a system having a RS operable to illuminate the scattering surface and/or volume portion from a plurality of radial angles of the RS's PV, and an imaging module configured to capture a Temporal Shadow Image(s) (TSI) from the scattering surface and/or volume portion (e.g., skin lesion) to generate Lateral Motion of Backscattered Light **(LMB),** Shadow Gradient Pattern Image **(SGPI)** and lateral phase flow Image **(LPFI),** thereby depicting the scattering surface and/or volume irregularities **(SViR)** portion. The SGPI imaging methods refers in an exemplary implementation, to the accumulated movements (or in other words collective changes in the LMB) among the TSIs captured in a given scan. The LPFI is a map of lateral phase flow (direction of the shadow to light movements) while changing PV in a given scan. The shadow-casting effect originates with the skewed collimated or partially collimated illumination of RS's PV. In the context of the disclosure, Collimation is considered collimation between 0 to about 20 ° (±5°).

In another exemplary implementation, provided herein is a system for in situ imaging of SViR in a skin lesion of a patient in need thereof, comprising: a housing defining longitudinal axis and a proximal base with transparent contact plate **(TCP)** for positioning over at least a portion of the skin lesion of the patient; at least one RS unit, operably coupled to the housing disposed distal to the TCP, sized and configured to illuminate in a variable manner at predetermined radial angles relative to the longitudinal axis of the housing; an imaging module sized and configured to capture TSIs each one at different angular state (angles: azimuth or altitude) of RS's PV; and optionally a polarizing and/or filter module disposed between the RS unit and the TCP and/or between the TCP and the lens.

In yet another exemplary implementation, the RS in the system is comprised of a plurality of RSs radially coupled to the housing, each RS configured to illuminate at predetermined timings, and wherein the timings configured to illuminate the lesion by varying the RS's PV angles, the system further comprising a communication module configured to maintain communication with a communication network; and a central processing module **(CPM),** in communication with the imaging module, each of the plurality of RS, an actuator, and the communication module, the CPM further comprises at least one processor in communication with a non-transitory memory storage device storing thereon a processor-readable media with a set of executable instructions, configured when executed, to cause the at least one processor to: using the plurality of RS, illuminate at least one portion of the scattering surface and/or volume (e.g., skin lesion) from a plurality of angles; capture TSI at each illumination angle; using the captured plurality of TSIs, generate LMB, SGPI and LPFI depicting the portion of the scattering surface and/or volume (e.g., skin lesion) SViR And optionally a polarizing and/or filter module disposed between the RS unit and the TCP and/or between the TCP and the lens.

According to the invention, the RS (either as a single RS, or as comprised of a plurality of RS's) is coupled to a rotatable annulus, the rotatable annulus configured to at least one of: rotate around the longitudinal axis, and translate axially along the longitudinal axis relative to the scattering surface and/or volume portion (e.g., the skin lesion), the rotatable annulus is operably coupled to an actuator, configured to selectively effect at least one of: rotate the annulus, translate the annulus relative to the scattering surface and/or volume portion in parallel with the TCP and around the longitudinal axis, the system further comprising: a communication module configured to maintain communication with a communication network; and a central processing module (CPM), in communication with the imaging module, the at least one RS, the actuator, and the communication module, the CPM further comprises at least one processor in communication with a non-transitory memory storage device storing thereon a processor-readable media with a set of executable instructions, configured when executed, to cause the at least one processor to: actuate the actuator to rotate the annulus. While rotating, capture a plurality of TSIs from the imaging module; using the captured TSIs to generate LMB, SGPI and LPFI depicting the SViR of the scattering surface and/or volume portion.

In yet another exemplary implementation, provided herein is a method of imaging SViR of a scattering surface and/or volume portion of a patient in need thereof, implementable in a system comprising: a housing defining longitudinal axis and a proximal base with a TCP for positioning over the scattering surface and/or volume portion of the patient; at least one RS operably coupled to the housing disposed distal to the base, sized and configured to emit radiation in a variable manner at a predetermined angle relative to the longitudinal axis of the housing; a lens disposed distal to the TCP; an imaging module sized and configured to capture a TSI due to each illumination of the RS; and optionally a polarizing and/or filter module disposed between at least one of: the RS and the TCP , and between the TCP and the lens, the method comprising: positioning the TCP over at least a portion of the skin lesion; illuminating the lesion using the RS from a plurality of radial angles; capturing an image (TSI) at each angle of illumination; and using the captured TSIs to generate LMB, SGPI and LPFI depicting the SViR of the scattering surface ab/or volume portion.

The systems, devices, methods and programs for generating LMB, SGPI and LPFI to characterize and depict SViR of the scattering surface and/or volume portion, and TSI's patterning behavior, will become apparent from the following detailed description when read in conjunction with the drawings, which are exemplary, not limiting, and wherein like elements are numbered alike in several figures.

### BRIEF DESCRIPTION OF THE DRAWINGS

For a better understanding of the methods and systems for SViR detection of a scattering surface ab/or volume portion (e.g., a skin lesion) utilizing LMB, SGPI and LPFI described herein, with regard to the exemplary implementation(s) thereof, reference is made to the accompanying drawings, in which like numerals designate corresponding elements or sections throughout and in which:

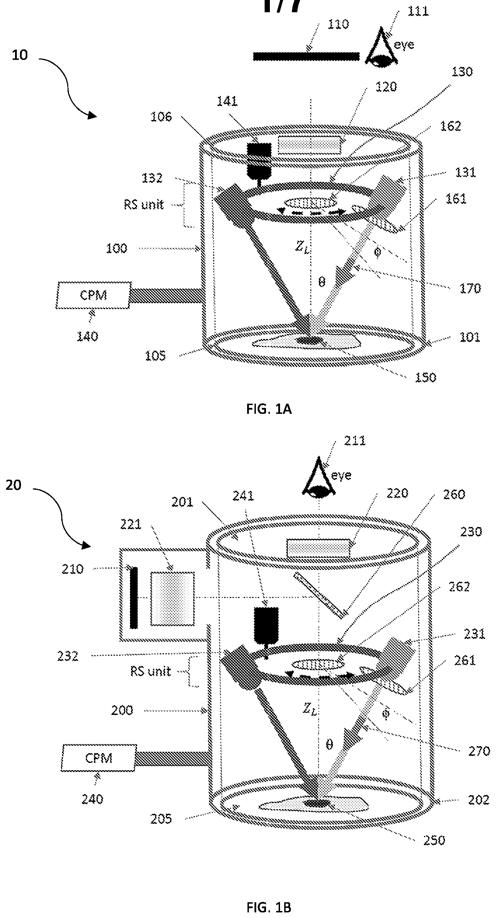

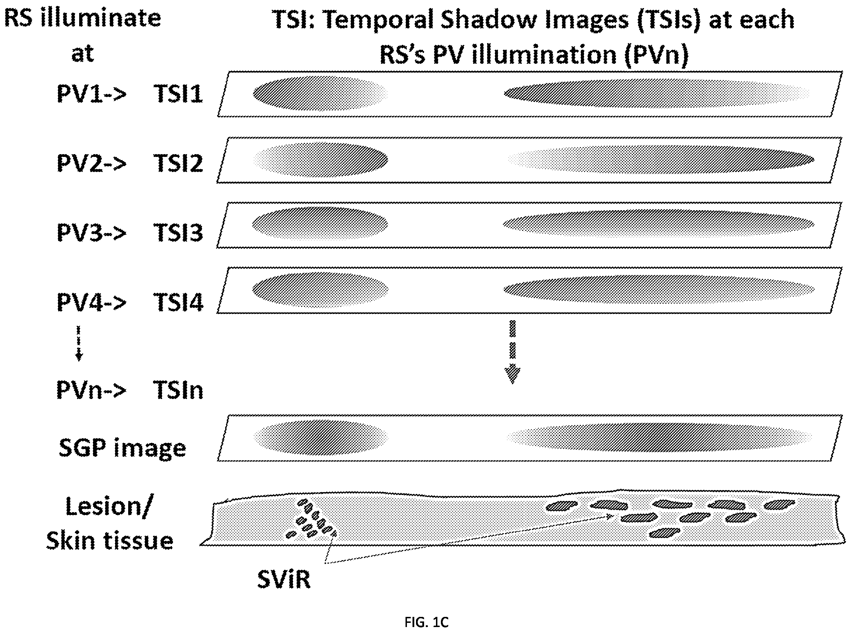

FIG. 1A, shows a schematic representation of a first configuration of the system with an observation TCP, with FIG. 1B showing a schematic representation of another configuration of the system with an observation TCP and a prism deflecting the image captured to an imaging module, while FIG. 1C, illustrates plurality of TSIs generated from differences in the RS illumination at various angles (PV angles, PV1..PVn, n-number of PV angle state).
FIG. 2, illustrating signals of two adjacent pixels in an image captured in an exemplary implementation by a digital camera demonstrating lateral intensity movement and a gradient between the pixels - over time or in other words between (and among) TSIs;

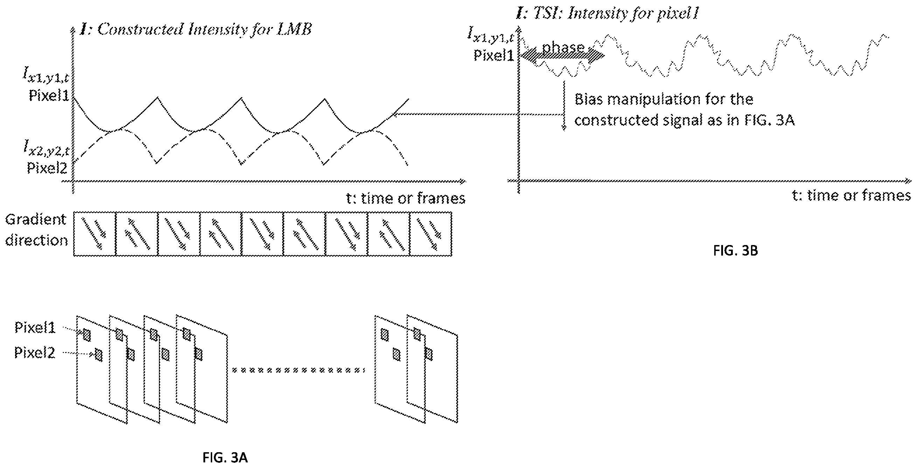

FIG. 3, illustrating the intensity movement between two adjacent pixels in a digital camera (over TSIs) following Pixel1 and Pixel2 in FIG. 2, captured at each time PV angle changes; quantified gradient is shown to illustrate the intensity lateral movements between Pixel1 to Pixel2 and vise versa;

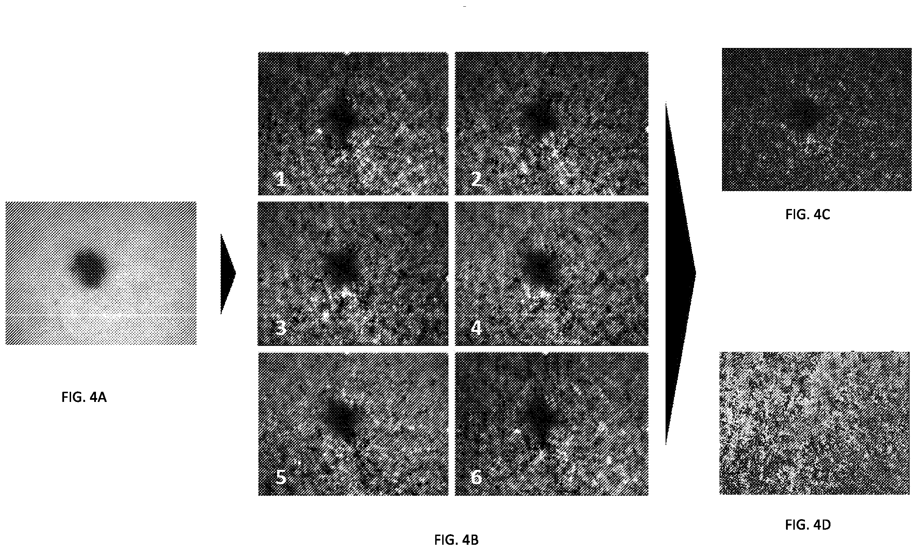

FIG. 4A, depicts an image captured by a dermascope of a benign lesion, FIG. 4B, depicting the LMB images captured at 6 different angles, with FIG. 4C depicting the cumulative SGPI of FIG. 4B, and FIG. 4D, depicting the LFP operation on FIG. 4B, providing the SViR of the benign lesion depicted in FIG. 4A;

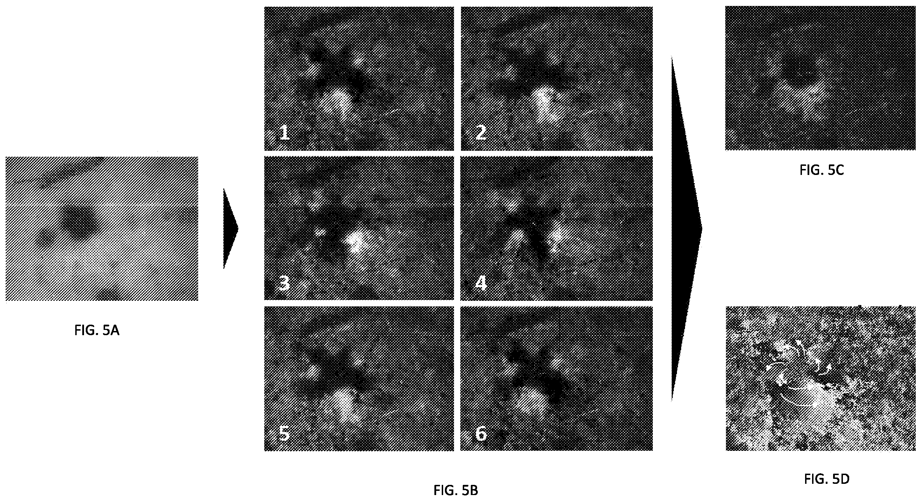

FIG. 5A, depicts an image captured by a dermascope of an in-situ (stage 0) malignant melanoma, FIG. 5B, depicting the LMB images captured at the same 6 different angles depicted in FIG. 5B, with FIG. 5C depicting the cumulative SGPI of FIG. 5B, and FIG. 5D, depicting the LFP operation on FIG. 5B, providing the SViR of the malignant lesion depicted in FIG. 5A : and

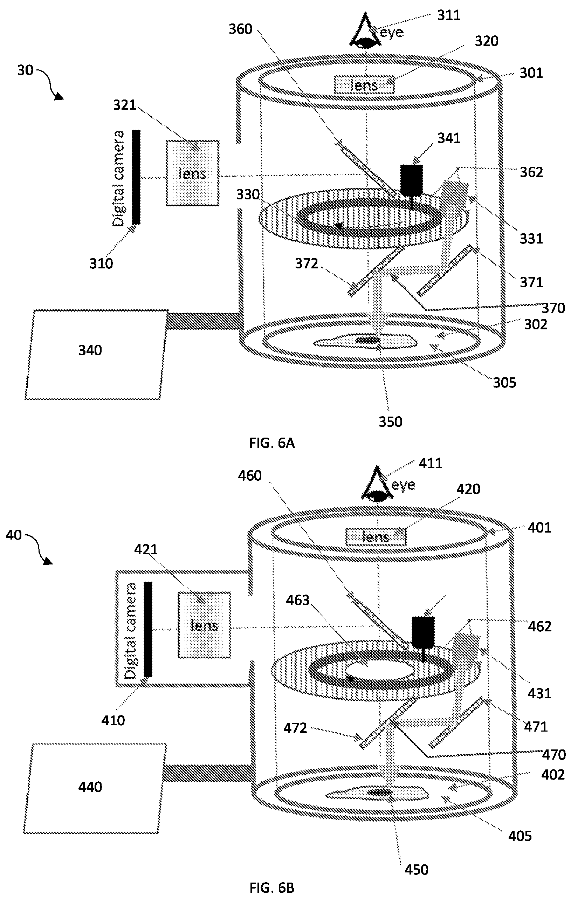

FIG. 6A, and 6B showing a schematic representation of a configuration of the system with pseudo-perpendicular illumination mirror (or semi mirror, or prism) with polarization disc and tilted mirror and a prism deflecting the image captured to an imaging module, without an analyzer in FIG. 6A, and with an analyzer in FIG. 6B.

### DETAILED DESCRIPTION

Provided herein are exemplary implementation(s) of device, systems and methods for the non-invasive characterization of skin lesions. In addition, the device, systems and methods provided herein may be used to diagnose malignancy of the skin lesion, lead to early detection of malignancy and provide for continuous monitoring of changes in the lesions surface and/or volume irregularities **(SViR)** over time, which may indicate transitions from benign to malignant (atypical, dysplastic, neoplastic and in between). Lateral motions of Backscattered light **(LMB),** Shadow Gradient Pattern Image **(SGPI)** and Lateral Phase Flow Image **(LPFI)** methods are a way to represent or resemble the SViR in none deterministic way. Hence it gives general information on the SViR of a lesion. But enabling to have a follow-up of the SViR can save lives by catching the lesion deterioration into malignancy at early stage.

Dermascopy is a form of noninvasive diagnostic technique that aims to characterize dermatopathology through the visualization of morphological irregularities and their changes that are not discernible by examination with the naked eye. Several lesion scoring systems are typically used to characterize and diagnose skin lesion as an aid to diagnose, characterize, and determine referral or biopsy. Common examples are the ABCD-E rule, the 7-point checklist (weighted and unweighted 7PCL), three-point checklist (3PCL), and the Menzies method. All of these methods relay on irregular color or irregular color distribution, in the ABCD rule; irregular pigmentation and border in the weighted and unweighted 7PCL; the atypical network (referring to a network of increased variability in the color, thickness and spacing of the lines of the network, asymmetrically distributed, and grayscale colored in the 3CPL; or symmetry of pattern structures through the center of the lesion in the Menzies method.

It stands to reason then, that devices, systems and methods for imaging the SViR using LMB, SGPI and LPFI will be advantageous in characterizing the lesion for further diagnosis, since it is not subjected to color but more aiming to the irregularities which are the precursor to pigmentation. SViR represents the degradation from atypical lesion to dysplastic and neoplastic - to be aware to the changes in SViR over time can be life-saving!. Accordingly and in an exemplary implementation, provided herein is a system for in situ characterizing SViR of a skin lesion in a patient in need thereof, comprising: a housing defining longitudinal axis and a proximal base with a TCP for positioning over at least a portion of the skin lesion of the patient; at least one RS (referring to an illumination device comprising one or more light sources, a power supply, and a controller or switch) operably coupled to the housing disposed distal to the base, sized and configured to illuminate in a variable manner at a predetermined angles relative to the longitudinal axis of the housing; a lens disposed distal to the TCP; an imaging module disposed distal to the TCP, sized and configured to capture the TSIs generated by alternations in Poynting Vector **(PV,** referring to the energy flux density (energy in Jouls per unit area in mm² per unit time in sec.) of the RSs in various angles (in this case); and optionally a polarizing and/or filter module disposed between the RS and the TCP and/or between the TCP and the lens.

In the context of the disclosure, capturing one image for each state of PV will be named Temporal Shadow Image (**TSI**). First part of the method is to capture multiple TSIs and extract shadow and light lateral movements. Then a computerize process generates a single image of cumulative SGPI of the scatterer's SViR for the scanned portion. PV alternations can also be revealed by monitoring the LPFI - which is the direction of the lateral changes. LMB originate by altering the PV state. The cumulative LMB images are used to generate the SGPI and the LPFI. Thus, SGPI qualitatively depicts the scatterer's SViR. LMB and LPFI are the characteristics due to the information concealed in the movement of light at the camera detector during altering PV (or RS).

In the context of the disclosure, the term "shadow" refers to the partial shadow-casting caused by all optical phenomena in the scattering surface and/or volume portion (reflection, absorption, depolarization... etc.) resulting from the skewed illumination (as alternation of PV). As an extreme example, just how the sun projects different shadows upon a tower. One can imagine the tower become more and more transparent (considering the tower as SViR in the air), the shadow starts to be distorted, and if we collect all of these distortions around the various angles, we can conclude the existence of the transparent tower. Also, the term shadow indicates a condition relating to an irradiation region or lack of it, where illumination from an RS can directly reach the region of interest (e.g., a lesion's border), but illumination from other state of PV, or other radial angles of the same RS cannot directly reach the given region because of a SViR partially being opaque.

Likewise, the term "radiation" or "illumination" are used herein to include the entire spectrum of electromagnetic radiation and any radiation useful to interact with the lesion at various depths is consistent with the present disclosure.

In certain configurations, the RS unit can be comprised of a single RS (illuminator) providing collimated illumination onto the surface of the scatterer (as for skin or lesion), and the change in illumination can be executed by manually or automatically (in other words, without user intervention) rotating the housing to provide variable angles (e.g. radial). The collimation of light should be understood broadly in the context of the disclosure, without limiting its scope. The term "collimation" and its derivatives, e.g., "collimated" and the like, generally refers to reducing the divergence of light decoupled from the illuminator(s) in one or more dimensions and/or planes as compared to the case when the divergence of light exiting the illuminator(s) is primarily defined by the broad angular distribution of light propagating in the illuminator by means of the optical properties of the reflectors coupled to the illuminator(s).

In the context of the disclosure, the term "Radial angle" refers to the angle along the Radial Path around the scanned portion of the lesion (around the longitudinal axis). Additionally, or alternatively, in certain configurations, varying the radial angle can be achieved by a plurality of RS radially (generating variations in PV) coupled to the housing, each RS configured to illuminate at predetermined timing configuration, and wherein the timing configuration configured to illuminate the lesion from varying radial angles. In other words, a series of RS having either the same or different spectrum emission are operably coupled to the housing, forming a circular ring around the longitudinal axis defined by the housing, the RSs (forming the RS complex in one exemplary implementation) being further coupled to an integrated circuit sized, adapted and configured to time and power each light source, thereby providing illumination from a variety of radial angles. In certain implementations, the plurality of RS can be positioned at variable heights relative to the proximal base, thus providing variable illumination angles (*θ,ϕ*, see e.g., FIG. 1).

In certain other configurations, varying the angle can be achieved by incorporating an annulus within the housing, the annulus being rotatable and coupled to the housing and to an actuator, configured to selectively affect at least one of: rotate the annulus, translate the annulus relative to the skin lesion along the longitudinal axis, the rotatable annulus operational to at least one of: rotate around the longitudinal axis, and translate along the longitudinal axis relative to the skin lesion. In other words, in addition to illuminating the lesion from variable radial angles affected by rotating the annulus, the actuator can further slide the annulus up and/or down relative to the lesion, thus providing different illumination angles thus generating different shadow.

As used herein, the term "actuator" should be understood to encompass a wide variety of devices, motor, including spring, hydraulic, pneumatic, or combinations of spring, hydraulic and pneumatic actuators. The term "actuator" may also encompass a portion of a robotic system configured to apply the requisite actuation motion to the annulus. The actuator (see e.g., FIG. 1A, element 141) is operative to actuate the annulus to rotate relative to the housing. As used herein, the term "actuator" broadly refers to any device used to directly or indirectly move another device (and, thus, is operative to actuate (or, in some situations, rotate) the annulus 130. An actuator can take any suitable form, including, but not limited to, a motor, a linear actuator, a piston, a rack-and-pinion device, and systems that use hydraulic fluids. An actuator "actuates" the annulus by moving it in any suitable manner, including, but not limited to at least one of: rotating, vibrating, and linearly moving the annulus 130. While not required in all implementations and configurations, the actuator can also be operative to track the position of the at least one RS. Furthermore, in some implementations, the actuator actuates the annulus (i.e., actuates in any suitable form of actuation). In other implementations, the actuator translates the annulus up and/or down relative to the proximal base of the housing. Accordingly, the term "actuate" in the claims should not be limited to rotate. Also, actuators other than a motor (such as those listed above) can be used to rotate the first reflective surface. Accordingly, the phrase "actuator operative to rotate" in the claims should not be limited to a motor. The angular changes shall be defined as the changes in latitude and longitude of at least 1 degree (ϕ, θ in FIG. 1-2).

As indicated, the lens present in the housing, can be a removable and be configured to filter visible light spectrum, providing incident light (*IL*, see e.g., FIG. 1A) at a predetermined wavelength, for example, between about 350 nm and about 1000 nm. The term "filter" refers to a material (e.g., the lens composition) that prevents the transmission of some wavelengths region to go pass through the filter material, regardless of the nature of the prevention mechanism, thus provide additional information on the SViR.

To acquire a TSI of a portion of the scattering surface and/or volume (e.g., skin lesion), it is illuminated in certain configurations with illumination that exhibits the appropriate intensity, spread, wavelength and timing for acquisition. Depending upon the features being imaged (e.g., sort of SViR of a lesion) and the relative angle of the imaging module's (e.g., camera) axis to the features, the type and characteristics of illumination employed can vary widely. For example, some features (e.g. lesion thickness) can be best imaged using low *θ* angle illumination, while other features (e.g. border) are typically best imaged using direct, high *θ* angle illumination. Likewise, the wavelength and spectrum of illumination can vary depending on the nature of the features-that is, some features are best illuminated in the visible range, while others may be enhanced using infrared (IR) light.

Each RS illuminator can comprise a light-emitting diode (LED), configured to emit electromagnetic radiation at a predetermined wavelength and intensity. The wavelength can be the whole visible spectrum (e.g., incandescent, white LED, or white organic light-emitting diode, WOLED) with a filter lens that will allow a predetermined wavelength to be emitted from the lens. RS can also be controlled to a potentiometer or PWM (pulse width modulator) and be configured to selectively operate as a strobe (in other words, provide flashes of light intensity emission for a short duration).

Likewise, for some applications it is desirable to employ polarized light and/or filter in the light path between RS and lesion and/or between lesion and camera. Certain implementations disclosed can comprise a ring illumination arrangement consisting of one or more rows of that encircle the lens. These illuminators can be external and remote from the camera housing, but "internal" in other configurations, being fixed in place on housing above the TCP. One form of ring illuminator, which surrounds the camera lens, is constructed in certain implementations on a circuit board that contains a predetermined number of lighting elements in one or more circles around the lens. The lighting elements on a typical illuminator are LEDs, but other types of light sources (e.g. xenon strobes, incandescent, laser, fluorescent light source, etc.) can also be employed, either as an alternative to LEDs or in addition to LEDs.

Accordingly, the term RS or "illuminator" as used in the context of the disclosure, shall refer generally to internal or external illuminators that are controlled by the CPM. Also, as used herein, the term "processor" in relation to the imaging module shall refer to one or more processing components that are generally contained within the imaging module unit, but that can be (at least in part) located remote from the imaging module unit enclosure and that carry out the illustrative functions described herein. These functions include control of the RS and settings that are dependent on the RS unit's parameters. Other image processing, camera-control functions and control on the actuator can also be performed by the "processor."

Furthermore, it is noted that the term "imaging module" as used herein means a unit that includes a plurality of built-in image and/or optic sensors and outputs electrical signals, which have been obtained through photoelectric conversion, as an image, while the term "module" refers to software, hardware, for example, at least one processor, or a combination thereof that is programmed with instructions for carrying an algorithm or method steps as disclosed herein. The modules described herein may communicate through a wired connection, for example, a hardwired connections, a local area network, or the modules may communicate wirelessly. The imaging module may comprise camera (analog to digital detector) like charge coupled devices (CCDs) or metal-oxide semiconductor (CMOS) or as such. TSI is acquired by the imaging module that can comprise a digital frame of the camera, where the field of view (FOV) can be predetermined by, for example, the camera size with its optics and the axial distance from the lesion surface (the TCP). The term "digital camera" refers to implementations to a digital still camera or a digital video recorder. The digital camera can comprise an image capturing unit or module, a capture controlling module, a processing unit like micro controller, field-programmable gate array (FPGA), or local processor (which can be the same or separate from the central processing module).

The transmission at the various wavelengths is configured each to penetrate the surface of the lesion or skin to different depth, thus providing different TSI and different information. Generally speaking, in the optical and near infra-red (NIR) region the shorter the wavelength the less penetration, hence different shadow in the TSI due to differences in scattering, absorption, refraction and reflection.

As illustrated in FIG. 1B, in certain configuration, the device used in the system disclosed further comprising: a transparent window as TCP coupled to the housing, sized and configured to allow direct observation of the skin lesion by a user (e.g., a physician, care provider, the patient etc.); and a prismatic element (referring to an optical prism, mirror, partial mirror), sized and configured to divert lesions backscattered light toward the imaging module.

As indicated, the devices used as part of the systems disclosed herein, use an RS unit, comprised of a plurality of RS illuminators having maximum emission wavelength that is the same or different, arranged in certain configurations as a circle operably coupled to at least one of: the housing, the annulus, and around the lens of the imaging module. Controlling the illumination behavior can be done by a switch (as described hereinabove), or at least one processor that is in communication with the imaging module and the CPM. In these implementations, the system can further comprise: a communication module configured to maintain communication with a communication network; and a CPM, in communication with the imaging module, the at least one illuminator, and the actuator. The communication module and the CPM comprises at least one processor in communication with a non-transitory memory storage device storing thereon a processor-readable media with a set of executable instructions, configured when executed, to cause the at least one processor to: using the plurality of RS' illuminators, illuminate the lesion from a plurality of angles; capture an image at each illumination angle, each image providing a TSI (see e.g., FIG. 1C, FIG. 2, FIG. 3, bottom); using the captured plurality of TSIs, calculate the LMB by constructing the signal for each pixel in a periodic way using the signal phase (in every pixel) and eliminating or modifying its bias. (the scan itself don't need to be periodic). It generates the lateral movements (LMB). Then calculate the total cumulative LMB as one image of SGPI and LPFI depict the SViR of the scattering surface and/or volume portion (skin lesion in this example) (see e.g., FIG. 1C bottom and FIG.s 4B-4D).

Turning now to FIG.s 4A-D, and 5A-D, where FIG. 4A-C illustrate a study case images of the SViR of a benign lesion as calculated by LMB (FIG. 4B), and SGPI (FIG. 4C), depicting a dermascope-captured lesion in FIG. 4A, being compared with skin cancer FIG. 5A showing a lesion with high surface variability as can be shown in its LMB (FIG. 5B) and its SViR imaging (FIG. 5C). Each of the six images shown in their LMB stems from different state of PV. Nevertheless, the dermoscopy images FIG.4A and FIG. 5A look very similar, a prominent change in the SViR can show its differences in the underneath deformation (SViR). The benign case (FIG. 4C) revealed to be with random patterns and the skin cancer (FIG. 5C) looks like an explosion from the middle outward. Figures FIG. 4D and FIG. 5D are the LPFI of the lateral phase indicate the lateral movements of the LMB. The directions of the arrows go from dark to bright (phase of -3.1415 to 3.1415).

In the context of the disclosure, the term "random", is not intended necessarily to denote pure randomness in the theoretical sense. A signal such as the LPFI, does not need to be purely random in order for it to be sufficiently random to be useful. The degree of randomness, or unpredictability, depends on the particular application of use. Therefore, the term "random" will be used herein to indicate a degree of unpredictability or randomness sufficient for use as a predictor of malignancy. Furthermore, and in reference to FIG.s 2, 3A and 3B, the term "unpredictability", as that term is used herein, is intended to mean that, given a perfect knowledge of the circuit and of the waveform of a signal up to a time "t", the value of the signal within some limit of precision at time "t+Δt" cannot be predicted. As the odds of a prediction being wrong approach 50% each time a prediction is made, the result can be regarded as a random bit string (captured in the randomness illustrated in FIG. 4D.

Using LMB, SGPI and LPFI methods, physicians will have new tool to make a more educated decision about the state of the tissue. As illustrated in comparing FIG.s 4C, 4D, 5C and, 5D, it is evident that random patterns imply a healthy tissue (or lesion) and directional (or otherwise, non-random) patterns, typically resembling a nimbus cloud or a burst imply dysplasm of the lesion, which may be a precursor of neoplasm (cancer).

As indicated, FIG. 4B shows six LMB images (constructed from TSIs using signal phase and modified bias) at six PV state illustrating the LMB effect of illuminating in various angles of RS (PV angles) on shadow-casting and resulting in the SGPI as shown in FIG. 4C. For healthy tissue the patterns are random compared with FIG. 5B-C which is show distorted lesion by directional patterns. FIG. 5B are six LMB images (constructed from TSIs using signal phase and modified bias) illustrating the LMB effect of illuminating in various angles of RS (e.g., PV angles), its result is the SGPI that indicates directional patterns as shown in FIG. 5C for neoplasm (skin cancer). SGPI as will be further explained is the accumulated changes (lateral gradient and/or phase) between the LMB images, laterally and in time as shown in FIG. 3. Using LMB , SGPI and LPFI methods physicians will have new tool to make a better decision about the state of the tissue. When the pattern is random it mostly implies on a healthy tissue (or lesion) and when the patterns are directional, mostly as nimbus clouds or burst of an explosion it depicts high deformations as in the case of dysplasm or neoplasm of the lesion which may be a precursor of cancer or already cancer.

In an exemplary implementation, the systems disclosed herein are used to implement the methods disclosed. Therefore, provided herein is a method of imaging and characterizing SViR of a scattering surface and/or volume portion (e.g., a skin lesion), implementable in a system comprising: a housing defining longitudinal axis and a proximal base with an TCP (coupled at the proximal base) for positioning over at least a portion of the skin lesion of the patient; at least one RS unit, operably coupled to the housing disposed distal to the base, sized and configured to emit radiation in a variable pattern at a predetermined radial angle relative to the longitudinal axis of the housing; a lens disposed distal to the RS unit; an imaging module sized and configured to capture a shadow pattern (due to phase extraction and bias elimination from TSIs to generate LMB images, FIG. 3B) from the transmission of the RS unit; and optionally a polarizing module disposed between the RS and the lens, the method comprising: positioning the TCP over at least a portion of the skin lesion; radiating the lesion using the RS unit from a plurality of radial angles; capturing an image at each radial radiation angle or polarization, each image providing a TSI; and using the captured images, generating LMB, SGPI and LPFI that depict the skin lesion's SViR. In certain implementations, where the RS unit comprises a plurality of illuminators (with the same and/or different wavelengths), the at least one of: the predetermined angle of at least one illuminator, and the distance between the RS unit and the skin lesion (in other words, the translation along the longitudinal axis (see e.g., FIG.s 1A, 1B), is selectively variable, and the method further comprises capturing a plurality of images at the same radial angle, however, at different axial distance from the skin lesion, and/or different illuminator angle *θ* (see e.g., FIG. 1A, 1B).

In an exemplary implementation, the step of generating the LMB, SGPI and LPFI of the skin lesion, comprises: generating a plurality of images (or a clip), each image (or frame) being of a single TSI per rotation step at a given axial distance (e.g., along *Z_{L}*) over the skin lesion; combining the plurality of TSI's to form LMB (see e.g., FIG. 2), SGPI (see e.g., FIG. 4C, 5C) and LPFI (See e.g., FIG.s 4D, 5D) depicting the SViR of the scattering surface ab/or volume portion (for example, the skin lesion) associated with the skin lesion irregularities. In other words, the cumulative SGPI is generated by extracting the LMB from TSI's changes over the frames (or image, each frame is actually a TSI at different PV state of illumination), (angles: azimuth or altitude), see e.g., FIG.s 1A, 1B) during the capturing of plurality of images (TSIs); the changes can be interpreted using in an exemplary implementation, the phase changes of the signal for each pixel and its bias (see e.g., FIG.s 3A, 3B), cumulative lateral gradient, spatial derivatives or mathematical transforms (spatial and periodic) to analyze the signals of the TSIs and LMB. The cumulative lateral movements in the LMB are determined to be SGPI and is associated with the characteristic SViR of skin lesion as shown in FIG. 1C, bottom (and FIG.s 4C, 5C).

A more complete understanding of the components, methods, and devices disclosed herein can be obtained by reference to the accompanying drawings. These figures (also referred to herein as "FIG.") are merely schematic representations based on convenience and the ease of demonstrating the present disclosure, and are, therefore, not intended to indicate relative size and dimensions of the devices or components thereof, their relative size relationship and/or to define or limit the scope of the exemplary implementation(s). Although specific terms are used in the following description for the sake of clarity, these terms are intended to refer only to the particular structure of the exemplary implementation(s) selected for illustration in the drawings, and are not intended to define or limit the scope of the disclosure. In the drawings and the following description below, it is to be understood that like numeric designations refer to components of like function. Likewise, cross sections are referred to on normal orthogonal coordinate system having XYZ axis, such that Y axis refers to front-to-back, X axis refers to side-to-side, and Z axis refers to up-and-down. Coordinates illustrating the RS movements are cylindrical, azimuth - θ*,* latitude - ϕ, height - *Z*_{L}.

Turning now to FIG. 1A illustrating a schematic representation of a first configuration of system 10 with camera as an optional replacement of the direct looking by eye, while FIG. 1B showing a schematic representation of another configuration of basic system 20 as in FIG. 1A but with a camera coupled radially, using deflector as a prism, partial mirror, or a mirror. As illustrated in FIG. 1A, Basic system 10 comprises housing 100 defining longitudinal axis *Z_{L}* and proximal base 101 with a TCP 105 for positioning over at least a portion of the skin lesion 150 of the patient; at least one RS unit. In the drawings one can find an example of two RSs 131, 132 but it can be any number of RS operably coupled to the housing 100 disposed distal to the proximal base 101, sized and configured to illuminate in a variable manner at a predetermined angle (latitude *θ*, azimuth *ϕ*, also can be illustrated by RS's PV direction 170) relative to the longitudinal axis *Z_{L}* of housing 100; lens 120 disposed distal to RS unit 131, 132; imaging module 110 disposed distal to RS unit 131, 132, sized and configured to capture a TSIs at every angle of RS illumination 131, 132; and optionally a polarizing or filter module 161 disposed between RS unit 131, 132 and TCP and/or as indicates 162 between TCP and imaging module 110 or eye of user 111 above the Transparent Observation Plate (TOP 106). Also illustrated in FIG. 1A, is central processing module (CPM 140), in communication with imaging module 110, the at least one illuminator (together forming an implementation of RS unit 131, 132 in this example), actuator 141, and the communication module (not shown, part of CPM 140), CPM 140 further comprises at least one processor (not shown, part of CPM 140), in communication with a non-transitory memory storage device (not shown, part of CPM 140), storing thereon a processor-readable media with a set of executable instructions, configured when executed, to cause the at least one processor to carry out the method steps disclosed herein. Also shown is annulus 130, of an exemplary implementation, coupled to housing 100 by being partially inserted in radial channel (not shown).

Turning now to FIG. 1B, illustrating a schematic representation of another exemplary implementation of system 20 with observation TOP 201. Exemplary implementation illustrates system 20 as comprising: housing 200 defining longitudinal axis *Z_{L}* and proximal base 202 with an TCP 205 for positioning over at least a portion of the skin lesion 250 of the patient; In the drawings one can find an example of two RSs 231, 232 but it can be any number of RS operably coupled to the housing 200 disposed distal to the proximal base 202, sized and configured to illuminate in a variable manner at a predetermined angle (latitude *θ*, azimuth *ϕ*, also can be illustrated by RS's Poynting Vector direction 270) relative to the longitudinal axis *Z_{L}* of housing 200; lens 220 disposed distal and transverse to RS unit (231, 232); imaging module 210 disposed distal to lens 221, sized and configured to capture a TSIs at every angle of RS illumination (231, 232); and optionally a polarizing module and/or filter 261, 262 disposed between RS unit (231, 232) and TCP and/or the TCP and lens 221, 220. System 20 further comprises: transparent observation plate **(TOP)** 201 situated above the prism inside housing 200, sized and configured to allow direct observation of the skin lesion 250 by the user 211; and prismatic element or mirror or partial mirror 260, sized and configured to divert the backscattered light from skin lesion 250 to lens 220 and 221. Also illustrated in FIG. 1B, is central processing module (CPM 240), in communication with imaging module 210, the at least one illuminator (231, 232 in this example together forming an implementation of RS unit), actuator 241, and the communication module (not shown, part of CPM 240), CPM 240 further comprises at least one processor (not shown, part of CPM 240), in communication with a non-transitory memory storage device (not shown, part of CPM 240), storing thereon a processor-readable media with a set of executable instructions, configured when executed, to cause the at least one processor to carry out the method steps disclosed herein. Also shown is annulus 230, of an exemplary implementation, coupled to housing 200 by being partially inserted in radial channel (not shown).

The device can further comprise a central processing module (CPM). According to various exemplary implementation(s) disclosed herein, the CPM can comprise a processing unit (typically including an arithmetic logic unit and a control unit) and a memory (also known as "registers," or/and Read Only Memory (ROM) and/or cache memory). The CPM may also comprise a non-transitory memory device, storing thereon an operating system and/or any further executable instructions and/or data configured to affect the function of the components described herein, according to the methods provided. The non-transitory memory device can be implemented within the CPM or externally. For example, the CPM may share a single memory coupled to a bus. As used herein, the term "memory" refers to any type of long term, short term, volatile, nonvolatile, or other storage devices and is not limited to any particular type of memory or number of memories, or type of media upon which memory is stored.

Two more system configurations are illustrated in FIG.s 6A, 6B, showing in FIG. 6A(6B) another exemplary implementation of system 30 with observation TOP 301 (401). Exemplary implementation illustrates system 30 (40) as comprising: housing 300 (400) defining longitudinal axis *Z_{L}* and proximal base 302 (402) with TCP 305 (405) for positioning over at least a portion of the skin lesion 350 (450) of the patient; At least one RSs 331 (431) is illustrated, operably coupled to the housing 300 (400) disposed distal to the proximal base 302 (402), sized and configured to illuminate in a variable manner at a pseudo-vertical angle (latitude (*θ*≈80°-89°), azimuth *ϕ*, ) as illustrated by RS's 331 (431) deflected Poynting Vector direction 370 (470) relative to the longitudinal axis *Z_{L}* of housing 300 (400) using at least one of a mirror, half - mirror, and prism arrangement 371,372 (471, 472) to modulate latitude angle *θ*, making it pseudo-vertical (in other words, about 90°). System 30 (40) also comprise lens 320 (420) disposed distal and transverse to RS unit 331 (431), with imaging module 310 (410) disposed distal to lens 321 (421), sized and configured to capture a TSIs at every pseudo-vertical angle θ of RS illumination 331 (431); and optionally a polarizing module and/or filter 361, (461) disposed between RS unit (331, 431) and TCP 305 (405) and/or an optional polarizing module and/or filter 362 (462) between the TCP 305 (405) and lens 321 (421). System 30 (40) further comprises: transparent observation plate **(TOP)** 301 (401) situated above prism (or semi-mirror, or mirror) 360 (460) inside housing 300 (400), sized and configured to allow direct observation of the skin lesion 350 (450) by the user 311 (411), sized and configured to divert the backscattered light from skin lesion 350 (450) to lenses 320 (420) and 321 (421) whereby system 40, further comprises analyzer 463. Also illustrated in FIG.s 6A, 6B, is central processing module CPM 340 (440), in communication with imaging module 310 (410), the at least one illuminator 331 (431), actuator 341 (441), and the communication module (not shown, part of CPM 340 (440)), CPM 340 (440) further comprises at least one processor, in communication with a non-transitory memory storage device, storing thereon a processor-readable media with a set of executable instructions, configured when executed, to cause the at least one processor to carry out the method steps disclosed herein. Also shown is annulus 330 (430), of an exemplary implementation of system 30 (40), coupled to housing 300 (400) by being partially inserted in radial channel (3000, (4000), not shown)

All ranges disclosed herein are inclusive of the endpoints, and the endpoints are independently combinable with each other. "Combination" is inclusive of blends, mixtures, alloys, reaction products, and the like. Furthermore, the terms "first," "second," and the like, herein do not denote any order, quantity, or importance, but rather are used to denote one element from another. The terms "a", "an" and "the" herein do not denote a limitation of quantity, and are to be construed to cover both the singular and the plural, unless otherwise indicated herein or clearly contradicted by context. The suffix "(s)" as used herein is intended to include both the singular and the plural of the term that it modifies, thereby including one or more of that term (e.g., the illuminator(s) includes one or more illuminator or emitter of radiation). Reference throughout the specification to "one exemplary implementation", "another exemplary implementation", "an exemplary implementation", and so forth, means that a particular element (e.g., feature, structure, and/or characteristic) described in connection with the exemplary implementation is included in at least one configuration described herein, and may or may not be present in other exemplary implementation(s). In addition, it is to be understood that the described elements may be combined in any suitable manner in the various exemplary implementation(s), and is provided as examples intended to provide an understanding of the disclosed technology.

The term "module" may refer to at least a self-contained component (unit, system or element) that is used in combination with other modules and/or components and/or a separate and distinct unit of hardware and/or software that may be used as a component in a device, such as a dermascope. The term "module" may also refer to at least a self-contained assembly of electronic components and circuitry, such as a circuit configured to control the actuator, wavelength, intensity and duration of an LED light that is installed as the RS unit or the imaging module. The term "module" may be used interchangeably with the term "unit."

In the context of the disclosure, the term "operable" means the system and/or the device and/or the program, or a certain element or step is fully functional, sized, adapted and calibrated, comprises elements for, and meets applicable operability requirements to perform a recited function when activated, coupled, implemented, actuated, effected, realized, or when an executable program is executed by at least one processor associated with the system and/or the device. In relation to systems and circuits, the term "operable" means the system and/or the circuit is fully functional and calibrated, comprises logic for, having the hardware and firmware necessary, as well as the circuitry for, and meets applicable operability requirements to perform a recited function when executed by at least one processor.

Accordingly and in an exemplary implementation, provided herein is a system for in situ characterizing irregularities of at least one of: a tissue surface, and a tissue volume in a patient in need thereof, comprising: a housing defining longitudinal axis and a proximal base with a transparent observation plate (TOP) for user's direct vision through lens and a transparent contact plate (TCP) for positioning over a portion of the at least one of: the tissue surface, and the tissue volume; at least one radiation source (RS) unit operably coupled to the housing or to a rotating annulus disposed distal to the proximal base, the at least one RS sized and configured to illuminate in a variable manner the portion of the at least one of: the tissue surface, and the tissue volume, at a predetermined angles relative to the longitudinal axis (Z_{L}) of the housing; a lens disposed distal to the at least one RS unit; an imaging module disposed distal to the at least one RS unit, sized and configured to capture a Temporal Shadow Image (TSI) above the at least one of: the tissue surface, and the tissue volume; and Optionally a polarizing or filter module disposed between the at least one RS unit and TCP. Optionally a polarizing or filter module disposed between the TCP and imaging module. Optionally a polarizing or filter module disposed between the TCP and TOP, wherein (i) the at least one RS unit is configured to illuminate one spectral band or multiple spectral bands, (ii) the at least one RS unit comprises at least one illuminator operably coupled to the housing, operable to vary at least one of: angle of illumination (PV), intensity, and spectrum [to generate TSIs to calculate LMB, SGPI and LPFI], wherein (iii) the at least one illuminator is configured to emit collimated light at an angle of about between 0 ° and about 20 °, (iv) the at least one RS unit is coupled to a rotatable annulus, the rotatable annulus operable to perform at least one of: rotate around the longitudinal axis, and translate along the longitudinal axis relative to the portion of the at least one of: the tissue surface, and the tissue volume, (v) the rotatable annulus is operably coupled to an actuator, configured to selectively effect at least one of: annulus rotation, annulus translation relative to the portion of the at least one of: the tissue surface, and the tissue volume, along the longitudinal axis, wherein (vi) each illuminator separate from an adjacent illuminator by no less than 1°, wherein (vii) the TCP is coupled to the top of the housing, sized and configured to allow direct observation of the portion of the at least one of: the tissue surface, and the tissue volume, by a user, the system further comprising: a prismatic element, a mirror or partial mirror, sized and configured to divert light that returns back from the skin lesion to TOP and the imaging module, wherein the system (viii) further comprising: a communication module configured to maintain communication with a communication network; and a CPM, in communication with the imaging module, the at least one illuminator, the actuator, and the communication module, the CPM further comprises at least one processor in communication with a non-transitory memory storage device storing thereon a processor-readable media with a set of executable instructions, configured when executed, to cause the at least one processor to: using the plurality of illuminators, illuminate the lesion from a plurality state of a PV; capture an image at each PV state, each image configured to provide a single TSI, thereby providing a plurality of TSI's; using the captured plurality of TSI's, generate LMB images and a cumulative **SGPI** and LPFI, as well as (ix) a communication module configured to maintain communication with a communication network; and a CPM, in communication with the imaging module, the at least one illuminator, the actuator, and the communication module, the CPM further comprises at least one processor in communication with a non-transitory memory storage device storing thereon a processor-readable media with a set of executable instructions, configured when executed, to cause the at least one processor to: actuate the actuator to rotate the annulus; while rotating, capture a plurality of images from the imaging module, each image providing a single TSI; using the captured plurality of TSI's, generate LMB images, SGPI and LPFI, wherein (x) at least one of: the predetermined angles of at least one illuminator, and the distance between the at least one RS unit and the portion of the at least one of: the tissue surface, and the tissue volume, is selectively variable and wherein (xi) the portion of the at least one of: the tissue surface, and the tissue volume, is a skin lesion.

In another exemplary implementation, provided herein is a method of imaging and characterizing SViR of a portion of the at least one of: a tissue surface, and a tissue volume, of a patient in need thereof, implementable in a system comprising: a housing defining longitudinal axis and a proximal base with a TCP for positioning over at least a portion of the skin lesion of the patient; at least one RS unit operable to provide a plurality PV states of illuminators each operably coupled to the housing, disposed distal to the proximal base, sized and configured to illuminate in a variable manner at a predetermined illumination angles around the longitudinal axis of the housing; a lens disposed distal to the at least one RS unit; an imaging module sized and configured to capture TSIs for each PV state of the at least one RS unit; and optionally a polarizing or filter module disposed between the RS unit and the skin and/or between the skin and the lens at TCP and/or imaging module, the method comprising: positioning the TCP over the portion of the at least one of: the tissue surface, and the tissue volume; using the at least one RS unit at a plurality PV states, illuminate the portion of the at least one of: the tissue surface, and the tissue volume; capturing an image at each PV state, each image providing a single TSI; and using the captured TSI's, generating LMB images, SGPI and LPFI of the portion of the at least one of: the tissue surface, and the tissue volume, representing the SViR, wherein (xii) each illuminator is tilted at a predetermined angle, the angle being selectively variable and configured to collimate the radiation on the same area, and (xiii) is configured to collimate the emitted light to an angle of between 0° and about 20°, wherein (xiv) the system further comprises a rotatable annulus operably coupled to the housing, wherein the rotatable annulus is additionally operably coupled to an actuator, configured to selectively radially rotate the annulus and axially translate the rotatable annulus along the housing longitudinal axis, the method further comprising (xv) capturing a plurality of images at different axial distance from the portion of the at least one of: the tissue surface, and the tissue volume, wherein (xvi) the plurality of illuminators are radially coupled to the housing, each illuminator configured to illuminate at a predetermined timing configuration, and wherein the timing configuration configured to illuminate the lesion from varying radial angles, the system further comprising (xvii) a TCP coupled to the top of the housing, sized and configured to allow direct observation of the skin lesion by users; and a prismatic element, mirror or partial mirror, sized and configured to divert light that returns back from the skin lesion to the lenses, as well as (xviii) a communication module configured to maintain communication with a communication network, and a CPM, in communication with the imaging module, the at least one illuminator, the actuator, and the communication module, the CPM further comprises at least one processor in communication with a non-transitory memory storage device storing thereon a processor-readable media with a set of executable instructions, the method further comprising: actuating the actuator to at least one of: rotate the annulus arbitrarily, translate the annulus relative to the portion of the at least one of: the tissue surface, and the tissue volume, and vary the angle of the at least one illuminator; capturing a plurality of TSI's from the imaging module; using the captured plurality of TSI's, generating LMB images , SGPI and LPFI of the portion of the at least one of: the tissue surface, and the tissue volume, representing the SViR, wherein (xix) the step of generating the LMB images, SGPI and LPFI of the portion of the at least one of: the tissue surface, and the tissue volume, comprises capturing and rendering time-variant phase appearance image of the portion of the at least one of: the tissue surface, and the tissue volume, comprising (xx) comprises: extracting the phase of repeated signal or full period of the annular in each pixel of the camera; generating a plurality of images, each image being of a single image per PV state; and combining the plurality of images to form single image of phase map after bias manipulation to generate LMB, then SGPI and LPFI, wherein (xxi) the system further comprises a communication module configured to maintain communication with a communication network, and a CPM, in communication with the imaging module, the at least one illuminator, the actuator, and the communication module, the CPM further comprises at least one processor in communication with a non-transitory memory storage device storing thereon a processor-readable media with a set of executable instructions, the method further comprising: using the plurality of illuminators, illuminating the lesion from a plurality of radial PV's; capturing an image at each PV state, each image forms a single TSI; using the captured plurality of TSI's, generating the LMB, SGPI and LPFI, (xxii) further comprising comparing the LMB and/or a single image generated by SGPI and/or LPFI referring the portion of the at least one of: the tissue surface, and the tissue volume, obtained from the same patient on another occasion, and wherein (xxiii) the portion of the at least one of: the tissue surface, and the tissue volume, is a skin lesion.

## Claims

1. A system for in situ characterizing surface and/or volume irregularities (SViR) of a tissue surface, or a tissue volume in a patient in need thereof, comprising:
a. a housing (100) defining a longitudinal axis (Z_{L}) and a proximal base with a transparent observation plate (106) for user's direct vision through a lens and comprising a transparent contact plate (105) for positioning over a portion of the tissue surface, or the tissue volume;
b. at least one radiation source unit (131, 132) operably coupled to an annulus (130) disposed distal to the proximal base within the housing, the annulus (130) being rotatable around the longitudinal axis of the housing using an actuator, the at least one radiation source unit is sized and configured to illuminate in a variable manner the portion of the tissue surface, or the tissue volume, at predetermined angles relative to the longitudinal axis of the housing;
c. a lens (120) disposed distal to the at least one radiation source unit;
d. an imaging module disposed distal to the at least one radiation source unit, sized and configured to capture a plurality of Temporal Shadow Images above the tissue surface, or the tissue volume, from variable radial angles by rotating the annulus.

2. The system of claim 1, further comprising a polarizing and/or filter module disposed between at least one of the radiation source unit and the transparent contact plate, and the transparent contact plate and the lens.

3. The system of claim 1, wherein the at least one radiation source unit comprises at least one illuminator operably coupled to the housing, operable to selectively vary at least one of: angle, intensity, and spectrum of illumination.

4. The system of claim 3, wherein the at least one illuminator is configured to emit collimated light having divergence angle between 0 ° and about 20 °.

5. The system of claim 1, wherein the annulus is further operable to translate along the longitudinal axis relative to the portion of the tissue surface, or the tissue volume.

6. The system of claim 1, wherein the rotatable annulus is operably coupled to an actuator, configured to selectively effect at least one of: annulus rotation, annulus translation relative to the portion of the tissue surface, or the tissue volume, along the longitudinal axis.

7. The system of claim 3, further comprising:
a. a communication module configured to maintain communication with a communication network; and
b. a central processing module, in communication with the imaging module, the at least one illuminator, the actuator, and the communication module, the central processing module further comprises at least one processor in communication with a non-transitory memory storage device storing thereon a processor- readable media with a set of executable instructions, configured when executed, to cause the at least one processor to:
i. using the radiation source unit to illuminate the lesion from a plurality state of a Poynting Vector states;
ii. capture an image at each of said plurality of Poynting Vector states, each image configured to provide a single Temporal Shadow Images, thereby providing a plurality of Temporal Shadow Images;
iii. using the captured plurality of Temporal Shadow Images to generate Lateral Motion of Backscattering images, cumulative Shadow Gradient Pattern Image and Lateral Phase Flow Image.

8. The system of claims 3; further comprising:
a. a communication module configured to maintain communication with a communication network; and
b. a central processing module, in communication with the imaging module, the at least one illuminator, the actuator, and the communication module, the central processing module further comprises at least one processor in communication with a non-transitory memory storage device storing thereon a processor- readable media with a set of executable instructions, configured when executed, to cause the at least one processor to:
i. actuate the actuator to rotate the annulus;
ii. while rotating, capture a plurality of images from the imaging module, each image providing a single Temporal Shadow Image;
iii. using the captured plurality of Temporal Shadow Images to generate at least one of Lateral Motion of Backscattered Light images, Shadow Gradient Pattern Image and Lateral Phase Flow Image.

9. A method for imaging and characterizing surface and/or volume irregularities (SViR) of a portion of a tissue surface, or a tissue volume, using the system of claim 1, the method comprising:
a. positioning the transparent contact plate over the portion of the tissue surface, or the tissue volume;
b. using at least one radiation source radiation source unit for illuminating the portion of the tissue surface, or the tissue volume at a plurality Poynting vector states,;
c. capturing at least one image for each of the plurality of Poynting Vector states, each image providing a Temporal Shadow Image; and
d. using the captured Temporal Shadow Image and generating at least one of Lateral Motion of Backscattered Light Images, Shadow Gradient Pattern Image, and Lateral Phase Flow Image of the portion of the tissue surface, or the tissue volume, representing the SViR.

10. The method of claim 9, wherein each illuminator is tilted at a predetermined angle, the angle being selectively variable and configured to collimate the radiation on the same area.

11. The method of claim 9 or 10, wherein said using at least one radiation source unit comprises selectively rotating a rotatable annulus operably coupled to the housing and selectively translating the rotatable annulus along the housing longitudinal axis.

12. The method of claim 11, further comprising: capturing a plurality of images associated with a plurality of radiation sources at different axial distances along said longitudinal axis from the portion of the tissue surface, or the tissue volume.

13. The method of claim 11, further comprising:
a. illuminating said portion of sample or tissue through a transparent contact plate (TCP) configured for positioning over a portion of the at least one of: the tissue surface, and the tissue volume; and
b. collecting backscattered radiation reflected from said sample or tissue through said transparent contact plate using a prismatic element, mirror or partial mirror, configured to divert light that returns back from the skin lesion to the lenses.

14. The method of claim 11, further comprising rendering a time-variant phase appearance image of the portion of the tissue surface, or the tissue volume.

15. The method of claim 14, wherein said rendering time-variant phase appearance image of the portion of the tissue surface, or the tissue volume, comprises:
a. extracting for each pixel of the camera a phase of a repeated signal;
b. generating a plurality of images, each image corresponding to a single image per PV state; and
c. combining the plurality of images to form a single image of a phase map after bias manipulation to generate the at least one of Lateral Motion of Backscattered Light images, Shadow Gradient Pattern Image and Lateral Phase Flow Image.

## Patentansprüche

1. System zur In-situ-Charakterisierung von Oberflächen- und/oder Volumenunregelmäßigkeiten (SViR) einer Gewebeoberfläche oder eines Gewebevolumens bei einem Patienten, welcher dessen bedarf, umfassend:
a. ein Gehäuse (100), welches eine Longitudinalachse (Z_{L}) und eine proximale Basis mit einer transparenten Beobachtungsplatte (106) für eine direkte Sicht eines Benutzers durch eine Linse definiert und eine transparente Kontaktplatte (105) zum Positionieren über einem Abschnitt der Gewebeoberfläche oder dem Gewebevolumen umfasst;
b. mindestens eine Strahlungsquelleneinheit (131, 132), welche operativ mit einem Ring (130) gekoppelt ist, welcher distal zu der proximalen Basis innerhalb des Gehäuses angeordnet ist, wobei der Ring (130) unter Verwendung eines Aktuators um die Longitudinalachse des Gehäuses drehbar ist, wobei die mindestens eine Strahlungsquelleneinheit dazu dimensioniert und eingerichtet ist, den Abschnitt der Gewebeoberfläche oder des Gewebevolumens in einer variablen Weise in vorbestimmten Winkeln relativ zu der Longitudinalachse des Gehäuses zu beleuchten;
c. eine Linse (120), welche distal zu der mindestens einen Strahlungsquelleneinheit angeordnet ist;
d. ein Bildgebungsmodul, welches distal zu der mindestens einen Strahlungsquelleneinheit angeordnet ist und dazu dimensioniert und konfiguriert ist, eine Mehrzahl von Temporal-Shadow-Bildern über der Gewebeoberfläche oder dem Gewebevolumen aus variablen radialen Winkeln durch Drehen des Rings zu erfassen.

2. System nach Anspruch 1, ferner umfassend ein Polarisations- und/oder Filtermodul, welches zwischen mindestens einem aus der Strahlungsquelleneinheit und der transparenten Kontaktplatte, und der transparenten Kontaktplatte und der Linse angeordnet ist.

3. System nach Anspruch 1, wobei die mindestens eine Strahlungsquelleneinheit mindestens eine Beleuchtungseinrichtung umfasst, welcher operativ mit dem Gehäuse gekoppelt ist und dazu betriebsfähig ist, selektiv mindestens eines aus: Winkel, Intensität und Spektrum der Beleuchtung zu variieren.

4. System nach Anspruch 3, wobei die mindestens eine Beleuchtungseinrichtung dazu eingerichtet ist, kollimiertes Licht mit einem Divergenzwinkel zwischen 0° und etwa 20° zu emittieren.

5. System nach Anspruch 1, wobei der Ring ferner dazu betriebsfähig ist, sich entlang der Longitudinalachse relativ zu dem Abschnitt der Gewebeoberfläche oder des Gewebevolumens zu verschieben.

6. System nach Anspruch 1, wobei der drehbare Ring operativ mit einem Aktuator gekoppelt ist, welcher dazu eingerichtet ist, selektiv mindestens eines aus: Ringdrehung, Ringverschiebung relativ zu dem Abschnitt der Gewebeoberfläche oder des Gewebevolumens entlang der Longitudinalachse zu bewirken.

7. System nach Anspruch 3, ferner umfassend:
a. ein Kommunikationsmodul, welches dazu eingerichtet ist, eine Kommunikation mit einem Kommunikationsnetzwerk aufrechtzuerhalten; und
b. ein zentrales Verarbeitungsmodul, welches in Kommunikation mit dem Bildgebungsmodul, der mindestens einen Beleuchtungseinrichtung, dem Aktuator und dem Kommunikationsmodul steht, wobei das zentrale Verarbeitungsmodul ferner mindestens einen Prozessor umfasst, welcher in Kommunikation mit einer nichtvorübergehenden Speichervorrichtung steht, welche ein prozessorlesbares Medium mit einem Satz ausführbarer Befehle darauf speichert, welche dazu eingerichtet sind, bei Ausführung den mindestens einen Prozessor dazu zu veranlassen:
i. die Strahlungsquelleneinheit zu verwenden, um die Läsion aus einer Mehrzahl von Poynting-Vektor-Zuständen zu beleuchten;
ii. ein Bild bei jedem der Mehrzahl von Poynting-Vektor-Zuständen zu erfassen, wobei jedes Bild dazu eingerichtet ist, ein einzelnes Temporal-Shadow-Bild bereitzustellen, wodurch eine Mehrzahl von Temporal-Shadow-Bildern bereitgestellt wird;
iii. die erfasste Mehrzahl von Temporal-Shadow-Bildern zu verwenden, um Lateral-Motion-of-Backscattering-Bilder, ein kumulatives Shadow-Gradient-Pattern-Bild und ein Lateral-Phase-Flow-Bild zu erzeugen.

8. System nach Anspruch 3; ferner umfassend:
a. ein Kommunikationsmodul, welches dazu eingerichtet ist, eine Kommunikation mit einem Kommunikationsnetzwerk aufrechtzuerhalten; und
b. ein zentrales Verarbeitungsmodul, welches in Kommunikation mit dem Bildgebungsmodul, der mindestens einen Beleuchtungseinrichtung, dem Aktuator und dem Kommunikationsmodul steht, wobei das zentrale Verarbeitungsmodul ferner mindestens einen Prozessor umfasst, welcher in Kommunikation mit einer nichtvorrübergehenden Speichervorrichtung steht, welche ein prozessorlesbares Medium mit einem Satz ausführbarer Befehle darauf speichert, welche dazu eingerichtet sind, bei Ausführung den mindestens einen Prozessor dazu zu veranlassen:
i. den Aktuator zu betätigen, um den Ring zu drehen;
ii. während des Drehens eine Mehrzahl von Bildern von dem Bildgebungsmodul zu erfassen, wobei jedes Bild ein einzelnes Temporal-Shadow-Bild bereitstellt;
iii. die erfasste Mehrzahl von Temporal-Shadow-Bildern zu verwenden, um mindestens eines aus Lateral-Motion-of-Backscattered-Light-Bildern, einem Shadow-Gradient-Pattern-Bild und einem Lateral-Phase-Flow-Bild zu erzeugen.

9. Verfahren zum Abbilden und Charakterisieren von Oberflächen- und/oder Volumenunregelmäßigkeiten (SViR) eines Abschnitts einer Gewebeoberfläche oder eines Gewebevolumens unter Verwendung des Systems nach Anspruch 1, wobei das Verfahren umfasst:
a. Positionieren der transparenten Kontaktplatte über dem Abschnitt der Gewebeoberfläche oder des Gewebevolumens;
b. Verwenden mindestens einer Strahlungsquellenstrahlungsquelleneinheit zum Beleuchten des Abschnitts der Gewebeoberfläche oder des Gewebevolumens bei einer Mehrzahl von Poynting-Vektor-Zuständen;
c. Erfassen mindestens eines Bildes für jeden der Mehrzahl der Poynting-Vektor-Zustände, wobei jedes Bild ein Temporal-Shadow-Bild bereitstellt; und
d. Verwenden des erfassten Temporal-Shadow-Bilds und Erzeugen mindestens eines aus Lateral-Motion-of-Backscattered-Light-Bildern, einem Shadow-Gradient-Pattern-Bild und einem Lateral-Phase-Flow-Bild des Abschnitts der Gewebeoberfläche oder des Gewebevolumens, welche die SViR darstellen.

10. Verfahren nach Anspruch 9, wobei jede Beleuchtungseinrichtung in einem vorbestimmten Winkel geneigt ist, wobei der Winkel selektiv variierbar ist und dazu eingerichtet ist, die Strahlung auf denselben Bereich zu kollimieren.

11. Verfahren nach Anspruch 9 oder 10, wobei die Verwendung mindestens einer Strahlungsquelleneinheit selektives Drehen eines drehbaren Rings, welcher operativ mit dem Gehäuse gekoppelt ist, und selektives Verschieben des drehbaren Rings entlang der Longitudinalachse des Gehäuses umfasst.

12. Verfahren nach Anspruch 11, ferner umfassend: Erfassen einer Mehrzahl von Bildern, welche mit einer Mehrzahl von Strahlungsquellen in unterschiedlichen axialen Abständen entlang der Longitudinalachse von dem Abschnitt der Gewebeoberfläche oder dem Gewebevolumen assoziiert sind.

13. Verfahren nach Anspruch 11, ferner umfassend:
a. Beleuchten des Abschnitts der Probe oder des Gewebes durch eine transparente Kontaktplatte (TCP), welche dazu eingerichtet ist, über einem Abschnitt von mindestens einem aus der Gewebeoberfläche und dem Gewebevolumen positioniert zu werden; und
b. Sammeln der rückgestreuten Strahlung, welche von der Probe oder dem Gewebe durch die transparente Kontaktplatte reflektiert wird, unter Verwendung eines prismatischen Elements, Spiegels oder Teilspiegels, welche dazu eingerichtet sind, das von der Hautläsion zurückkehrende Licht zu den Linsen umzulenken.

14. Verfahren nach Anspruch 11, ferner umfassend Rendern eines zeitvarianten Phasenerscheinungsbilds des Abschnitts der Gewebeoberfläche oder des Gewebevolumens.

15. Verfahren nach Anspruch 14, wobei das Rendern des zeitvarianten Phasenerscheinungsbilds des Abschnitts der Gewebeoberfläche oder des Gewebevolumens umfasst:
a. Extrahieren einer Phase eines wiederholten Signals für jedes Pixel der Kamera;
b. Erzeugen einer Mehrzahl von Bildern, wobei jedes Bild einem einzelnen Bild pro PV-Zustand entspricht; und
c. Kombinieren der Mehrzahl von Bildern, um ein einzelnes Bild einer Phasenkarte nach einer Bias-Manipulation zu bilden, um mindestens eines aus Lateral Motion of Backscattered Light-Bildern, einem Shadow Gradient Pattern-Bild und einem Lateral Phase Flow-Bild zu erzeugen.

## Revendications

1. Système de caractérisation in situ d'irrégularités de surface et/ou de volume (SViR) d'une surface de tissu ou d'un volume de tissu chez un patient en ayant besoin, comportant :
a. un boîtier (100) définissant un axe longitudinal (Z_{L}) et une base proximale avec une plaque d'observation transparente (106) pour la vision directe de l'utilisateur à travers une lentille et comportant une plaque de contact transparente (105) pour le positionnement sur une partie de la surface de tissu ou du volume de tissu ;
b. au moins une unité (131, 132) de source de rayonnement couplée de manière fonctionnelle à un anneau (130) disposé de manière distale par rapport à la base proximale à l'intérieur du boîtier, l'anneau (130) pouvant tourner autour de l'axe longitudinal du boîtier à l'aide d'un actionneur, l'au moins une unité de source de rayonnement est dimensionnée et configurée pour éclairer de manière variable la partie de la surface de tissu ou du volume de tissu, à des angles prédéterminés par rapport à l'axe longitudinal du boîtier ;
c. une lentille (120) disposée de manière distale par rapport à l'au moins une unité de source de rayonnement ;
d. un module d'imagerie disposé de manière distale par rapport à l'au moins une unité de source de rayonnement, dimensionné et configuré pour capturer une pluralité d'images d'ombre temporelle au-dessus de la surface de tissu ou du volume de tissu, à partir d'angles radiaux variables en tournant l'anneau.

2. Système selon la revendication 1, comportant en outre un module de polarisation et/ou de filtrage disposé entre au moins l'une parmi l'unité de source de rayonnement et la plaque de contact transparente, et la plaque de contact transparente et la lentille.

3. Système selon la revendication 1, dans lequel l'au moins une unité de source de rayonnement comporte au moins un illuminateur couplé de manière fonctionnelle au boîtier, servant à faire varier sélectivement au moins l'un parmi : l'angle, l'intensité et le spectre d'éclairage.

4. Système selon la revendication 3, dans lequel l'au moins un illuminateur est configuré pour émettre une lumière collimatée ayant un angle de divergence entre 0° et environ 20°.

5. Système selon la revendication 1, dans lequel l'anneau peut en outre être translaté le long de l'axe longitudinal par rapport à la partie de la surface de tissu ou du volume de tissu.

6. Système selon la revendication 1, dans lequel l'anneau rotatif est couplé de manière fonctionnelle à un actionneur, configuré pour effectuer sélectivement au moins l'un parmi : la rotation de l'anneau, la translation de l'anneau par rapport à la partie de la surface de tissu ou du volume de tissu, le long de l'axe longitudinal.

7. Système selon la revendication 3, comportant en outre :
a. un module de communication configuré pour maintenir une communication avec un réseau de communication ; et
b. un module central de traitement, en communication avec le module d'imagerie, l'au moins un illuminateur, l'actionneur et le module de communication, le module central de traitement comporte en outre au moins un processeur en communication avec un dispositif de stockage à mémoire non transitoire stockant sur celui-ci un support lisible par processeur avec un ensemble d'instructions exécutables, configurées lorsqu'elles sont exécutées, pour amener l'au moins un processeur à :
i. utiliser l'unité de source de rayonnement pour éclairer la lésion à partir d'une pluralité d'états de vecteur de Poynting ;
ii. capturer une image à chacun de ladite pluralité d'états de vecteur de Poynting, chaque image étant configurée pour fournir une seule image d'ombre temporelle, pour ainsi fournir une pluralité d'images d'ombre temporelle ;
iii. utiliser la pluralité d'images d'ombre temporelle capturées pour générer des images de mouvement latéral de rétrodiffusion, une image de motif de gradient d'ombre cumulé et une image de flux de phase latéral.

8. Système selon la revendication 3, comportant en outre :
a. un module de communication configuré pour maintenir une communication avec un réseau de communication ; et
b. un module central de traitement, en communication avec le module d'imagerie, l'au moins un illuminateur, l'actionneur et le module de communication, le module central de traitement comporte en outre au moins un processeur en communication avec un dispositif de stockage à mémoire non transitoire stockant sur celui-ci un support lisible par processeur avec un ensemble d'instructions exécutables, configurées lorsqu'elles sont exécutées, pour amener l'au moins un processeur à :
i. actionner l'actionneur pour faire tourner l'anneau ;
ii. pendant la rotation, capturer une pluralité d'images à partir du module d'imagerie, chaque image fournissant une seule image d'ombre temporelle ;
iii. utiliser la pluralité d'images d'ombre temporelle capturées pour générer au moins l'une parmi des images de mouvement latéral de lumière rétrodiffusée, une image de motif de gradient d'ombre et une image de flux de phase latéral.

9. Procédé d'imagerie et de caractérisation d'irrégularités de surface et/ou de volume (SViR) d'une partie d'une surface de tissu ou d'un volume de tissu, à l'aide du système selon la revendication 1, le procédé comportant :
a. le positionnement de la plaque de contact transparente sur la partie de la surface de tissu ou du volume de tissu ;
b. l'utilisation d'au moins une unité de source de rayonnement pour éclairer la partie de la surface de tissu ou du volume de tissu à une pluralité d'états de vecteur de Poynting ;
c. la capture d'au moins une image pour chacun de la pluralité d'états de vecteur de Poynting, chaque image fournissant une image d'ombre temporelle ; et
d. l'utilisation de l'image d'ombre temporelle capturée et la génération d'au moins l'une parmi des images de mouvement latéral de lumière rétrodiffusée, une image de motif de gradient d'ombre et une image de flux de phase latéral de la partie de la surface de tissu ou du volume de tissu, représentant les SViR.

10. Procédé selon la revendication 9, dans lequel chaque illuminateur est incliné à un angle prédéterminé, l'angle étant sélectivement variable et configuré pour collimater le rayonnement sur la même zone.

11. Procédé selon la revendication 9 ou 10, dans lequel ladite utilisation d'au moins une unité de source de rayonnement comporte la rotation sélective d'un anneau rotatif couplé de manière fonctionnelle au boîtier et la translation sélective de l'anneau rotatif le long de l'axe longitudinal du boîtier.

12. Procédé selon la revendication 11, comportant en outre : la capture d'une pluralité d'images associées à une pluralité de sources de rayonnement à différentes distances axiales le long dudit axe longitudinal à partir de la partie de la surface de tissu ou du volume de tissu.

13. Procédé selon la revendication 11, comportant :
a. l'éclairage de ladite partie d'échantillon ou de tissu à travers une plaque de contact transparente (TCP) configurée pour être positionnée sur une partie de l'au moins un parmi : la surface de tissu, et le volume de tissu ; et
b. la collecte d'un rayonnement rétrodiffusé réfléchi par ledit échantillon ou tissu à travers ladite plaque de contact transparente à l'aide d'un élément prismatique, d'un miroir ou d'un miroir partiel, configuré pour dévier la lumière qui revient de la lésion cutanée vers les lentilles.

14. Procédé selon la revendication 11, comportant en outre le rendu d'une image d'aspect de phase variant dans le temps de la partie de la surface de tissu ou du volume de tissu.

15. Procédé selon la revendication 14, dans lequel ledit rendu d'une image d'aspect de phase variant dans le temps de la partie de la surface de tissu ou du volume de tissu comporte :
a. l'extraction pour chaque pixel de la caméra d'une phase d'un signal répété ;
b. la génération d'une pluralité d'images, chaque image correspondant à une seule image par état PV ; et
c. la combinaison de la pluralité d'images pour former une seule image d'une carte de phase après manipulation de biais pour générer l'au moins une parmi des images de mouvement latéral de lumière rétrodiffusée, une image de motif de gradient d'ombre et une image de flux de phase latéral.
